(19) **Europäisches Patentamt / European Patent Office / Office européen des brevets**

(11) **EP 4 168 781 B1**

(12) **FASCICULE DE BREVET EUROPEEN**

(45) Date de publication et mention de la délivrance du brevet:
**06.08.2025 Bulletin 2025/32**

(21) Numéro de dépôt: **21735253.3**

(22) Date de dépôt: **21.06.2021**

(51) Classification Internationale des Brevets (IPC):
**G01N 21/83** *(2006.01)* **G01N 21/51** *(2006.01)*
**G01N 21/59** *(2006.01)* **G01N 33/28** *(2006.01)*

(52) Classification Coopérative des Brevets (CPC):
**G01N 21/51; G01N 21/59; G01N 21/83; G01N 33/2823;** G01N 2201/0668

(86) Numéro de dépôt international:
**PCT/EP2021/066880**

(87) Numéro de publication internationale:
**WO 2021/259877 (30.12.2021 Gazette 2021/52)**

(54) **DISPOSITIF ET PROCÉDÉ DE DÉTECTION DU SEUIL DE FLOCULATION D'UN MILIEU COLLOÏDAL, NOTAMMENT D'UN MILIEU COMPRENANT DES ASPHALTÈNES, PAR ADDITION DE SOLVANT ALIPHATIQUE**

VORRICHTUNG UND VERFAHREN ZUR BESTIMMUNG DER FLOCKUNGSSCHWELLE EINES KOLLOIDALEN MEDIUMS, INSBESONDERE EINES ASPHALTEN-HALTIGEN MEDIUMS, DURCH ZUGABE EINES ALIPHATISCHEN LÖSUNGSMITTELS

DEVICE AND METHOD FOR DETECTING THE FLOCCULATION THRESHOLD OF A COLLOIDAL MEDIUM, IN PARTICULAR OF A MEDIUM COMPRISING ASPHALTENES, BY ADDING AN ALIPHATIC SOLVENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.06.2020 EP 20305689**

(43) Date de publication de la demande:
**26.04.2023 Bulletin 2023/17**

(73) Titulaire: **TOTALENERGIES ONETECH**
**92400 Courbevoie (FR)**

(72) Inventeurs:
• **AL FARRA, Ahmad**
**76600 LE HAVRE (FR)**
• **JOSE, Frédéric**
**64390 BARRAUTE CAMU (FR)**
• **FANTOU, Sandra**
**76610 LE HAVRE (FR)**
• **OLIVIER, Jérôme**
**76620 LE HAVRE (FR)**

(74) Mandataire: **Fédit-Loriot**
**22, rue du Général Foy**
**75008 Paris (FR)**

(56) Documents cités:
**WO-A2-2005/003754      US-A1- 2010 053 622**
**US-B2- 10 281 454**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE TECHNIQUE

L'invention a pour objet un dispositif de mesure du seuil de floculation d'un milieu colloïdal par addition de solvant aliphatique et un procédé de mesure du seuil de floculation d'un milieu colloïdal par addition de solvant aliphatique mis en œuvre au moyen dudit dispositif.

ETAT DE LA TECHNIQUE

Les produits pétroliers, et notamment les fiouls ou les résidus de distillation du pétrole, généralement appelés "Produits noirs" dans la profession, sont des systèmes colloïdaux constitués d'asphaltènes - c'est à dire de molécules lourdes très aromatiques possédant des chaînes latérales paraffiniques - qui sont dispersés (ou encore appelés "peptisés") sous forme de micelles dans une phase huileuse. Ces systèmes colloïdaux peuvent être déstabilisés plus ou moins facilement, par exemple par craquage thermique ou par dilution. Ainsi, dans une raffinerie, le procédé de conversion, appelé viscoréduction, peut conduire à une précipitation des asphaltènes sous l'effet des températures élevées du procédé (généralement supérieures à 400°C). De même, la constitution de mélanges contenant de tels systèmes colloïdaux peut générer une précipitation de ces asphaltènes par floculation, en particulier si l'environnement de dilution est du type paraffinique.

Il est donc nécessaire de connaître ou d'estimer les caractéristiques de ces asphaltènes dans les produits noirs, tel un produit pétrolier ou un mélange de produits hydrocarbonés, afin d'évaluer sa stabilité intrinsèque, ainsi que sa réserve de stabilité associée. En effet, plus la réserve de stabilité sera élevée, moins le produit noir sera sujet à des problèmes de précipitation des asphaltènes, ou de compatibilité par dilution avec d'autres espèces chimiques, notamment des bases paraffiniques.

On notera que les fiouls ou résidus de pétrole sont constitués d'une matrice malténique (résines + paraffines) et d'asphaltènes dispersés sous forme colloïdale. Les asphaltènes qui ont un caractère très aromatique sont insolubles avec les paraffines qui ont un caractère aliphatique. Pour qu'un résidu soit stable, il est nécessaire que les asphaltènes soient maintenus en suspension (ou dispersés ou peptisés) dans la matrice huileuse. La peptisation des asphaltènes est assurée par les résines qui ont à la fois un caractère aromatique et un caractère aliphatique. Lorsqu'un résidu a été déstabilisé, les asphaltènes floculent en s'agglomérant sous forme de grosses particules pouvant générer des bouchages de filtres présents dans les différentes unités de traitement, ou encore des détériorations de la métallurgie, par exemple les tuyauteries par encrassement qui conduit à une perte d'efficacité énergétique et de la capacité des pipes.

La caractéristique appelée valeur S (« S-value »), ou encore stabilité intrinsèque, par exemple d'un produit noir, est définie dans la profession ainsi que dans la norme ASTM D7157-18 (Révision 2018) par l'expression suivante : S=aromaticité des malténes/aromaticité des asphaltènes, soit encore S=So/(1-Sa), dans laquelle,

- So représente le pouvoir du milieu à solubiliser les asphaltènes, c'est à dire le caractère aromatique du milieu. Plus celui-ci sera aromatique, plus le So sera élevé.
- Sa est le caractère aromatique des asphaltènes.
- 1-Sa représente l'aromaticité du milieu nécessaire pour solubiliser les asphaltènes présents.

Si S>1, les asphaltènes sont peptisés et sont donc stables. S-1 représente la réserve de stabilité (plus cette réserve sera élevée, moins le produit noir sera sujet à des problèmes de précipitation ou de compatibilité).

La sévérité d'un choc thermique, tel que celui apporté par une distillation ou une viscoréduction agit directement sur l'aromaticité des asphaltènes puisque le craquage thermique provoque la coupure des chaînes alkylées et la condensation des asphaltènes. Les asphaltènes plus condensés et moins ramifiés (Sa plus faibles) vont avoir besoin d'un solvant plus puissant pour rester dispersés. Ainsi, la connaissance de la valeur de S, reliée à celle du Sa, va permettre de préciser les réglages des conditions de marche de l'unité concernée pour que celle-ci soit opérée sans risque de précipitation des asphaltènes, et par conséquent, pour répondre aux différentes exigences en matière de qualité de l'exploitant.

Par ailleurs, la connaissance des valeurs du pouvoir solvant So et du caractère aromatique des asphaltènes Sa est nécessaire pour optimiser le mélange des différents constituants des fiouls. C'est ainsi que, si on ajoute un fluxant (produit capable d'abaisser la viscosité d'un mélange) de pouvoir solvant faible à un produit noir, par exemple viscoréduit, et présentant des valeurs So élevée et Sa faible, on réduit la valeur du So du mélange, ce qui peut conduire à une déstabilisation du produit noir, et par conséquent à une floculation des asphaltènes, car les valeurs So et Sa résultantes seraient trop faibles pour satisfaire la relation S>1, c'est à dire la condition pour que les dites asphaltènes soient peptisées, donc stables.

De façon habituelle, on détermine au laboratoire les valeurs de S et Sa, puis par calcul So, d'un produit noir par une dilution étagée à l'aide d'un solvant paraffinique dudit produit noir, préalablement mélangé à un solvant aromatique. On note le moment où une floculation intervient. La mesure est répétée pour au moins un autre mélange avec un taux de dilution différent. On obtient ainsi des résultats qui permettent par corrélation linéaire d'obtenir les valeurs recherchées de S et Sa, puis d'en déduire par calcul So.

Expérimentalement, le seuil de floculation dans un mélange donné peut être décelé par l'intermédiaire de plusieurs sondes optiques fonctionnant dans l'infra-rouge (IR) ou le proche infra-rouge (NIR).

Par exemple, la technique décrite dans les brevets FR-A-2 596 522 ou US-A-4 628 204, de la société Texaco Belgium SA, permet de mesurer par IR le seuil de floculation d'une solution colloïdale pendant sa dilution. Cette mesure nécessite au préalable le bon choix de la sonde optique de mesure (il y a plusieurs sondes) en fonction de la nature et notamment de la présence des asphaltènes plus ou moins importante dans le produit noir à tester. En cas de mauvais choix de l'opérateur, il est alors nécessaire de procéder au nettoyage de l'appareillage, puis à une nouvelle préparation de l'échantillon pour une nouvelle mesure avec une autre sonde, ce qui induit une perte de temps pouvant être supérieure à une heure de temps opérateur, alors que le temps d'une analyse est d'environ 1H30 à 2H00, notamment si c'est le choix d'une sonde différente qui s'avère judicieux.

Un autre exemple est la méthode développée par la société Shell, en collaboration avec son partenaire néerlandais Zematra, fabricant d'appareils d'analyses. Cette méthode, dans laquelle la détection du seuil de floculation du milieu colloïdal s'effectue à l'aide d'une seule sonde, constituée d'une simple fibre optique entourée de verre, n'est malheureusement pas utilisable pour toute la gamme des produits noirs. En effet, le chauffage systématique de l'échantillon à 150°C, outre les problèmes de sécurité, peut provoquer, pour certains types de produits noirs, des dégradations préjudiciables à la mesure du seuil de floculation. Le temps d'une analyse, quant à lui, est relativement long puisque pouvant être supérieur à 5 heures.

Une autre méthode est également proposée pour mesurer la valeur de S sur les produits noirs avec un appareil "Porla", fabriqué par la société finlandaise FMS (Finnish Measurement Systems Ltd), et commercialisé par la société anglaise Med-Lab. Cet appareil utilise une cellule de mesure à circulation continue de l'échantillon à analyser avec une détection optique du seuil de floculation au moyen d'un prisme fonctionnant en réflexion totale. La gamme de mesure est très large et un résultat est toujours accessible, même avec des produits noirs dont le seuil de floculation est réputé difficile à mesurer. Toutefois, ces résultats sont obtenus après modifications des paramètres opératoires de la méthode, qui deviennent alors une fonction de la nature du produit, ce qui est inacceptable quand la gamme des produits à analyser est très variable, comme dans l'industrie du pétrole.

Le document DE3714755A1 décrit un dispositif de mesure de la taille de flocons (« flakes » en anglais) dans un fluide. Le dispositif présente un canal de mesure situé entre des éléments optiques qui peuvent être déplacés l'un par rapport à l'autre afin de régler l'espace les séparant. L'espace séparant les éléments optiques forme en outre un angle afin de retenir des flocons de dimensions différentes le long des éléments optiques. Les dimensions de cet espace sont ainsi choisies en fonction des dimensions des flocons à mesurer. Une mesure de la luminosité le long des éléments optiques permet de déterminer les dimensions des flocons retenus entre les deux éléments optiques. Il n'est pas décrit de mesure du seuil de floculation.

Le document US2010/053622A1 décrit un système d'analyse permettant de quantifier des inhomogénéités contenues dans un échantillon de fluide. Ce système utilise une lentille optique, par exemple un microscope, pour focaliser un rayon lumineux sur l'échantillon. A cet effet, le support d'échantillon peut être rapproché ou éloigné de la lentille afin de positionner l'échantillon dans un plan focal. L'intensité de la lumière transmise au travers de l'échantillon est mesurée par un détecteur situé du côté opposé à la lentille par rapport à l'échantillon. L'échantillon est déplacé selon un motif prédéterminé dans le plan focal afin que l'intensité lumineuse qui le traverse soit mesurée le long d'un chemin représentatif de l'échantillon. L'intensité lumineuse est fortement atténuée quand elle rencontre une particule, ce qui permet la caractérisation et la quantification des particules présentes dans l'échantillon. Le déplacement de l'échantillon a ainsi pour unique fonction la détection des particules.

Le document WO2005003754A2 décrit un appareil de dosage automatique pour déterminer l'incompatibilité de produits pétroliers. Le système de détection consiste en un spectromètre de transmission de lumière par fibre optique, le liquide à mesurer passant dans une cellule optique de 100$\mu$m d'épaisseur qui n'est pas détaillée. L'appareil est équipé d'un circuit relié à plusieurs réservoirs et pompes permettant l'introduction d'un produit pétrolier, d'un solvant aliphatique, d'un solvant aromatique et d'un solvant auxiliaire dans un récipient de mélange thermostaté utilisé pour le dosage. Le temps de mesure d'un échantillon est de 1 à 2 heures.

Il existe aujourd'hui une norme (ASTM D7157-18 -Révision 2018) pour la détermination des valeurs S, Sa, So, qui peut être mise en œuvre au moyen d'un dispositif et d'un procédé décrits dans le document EP1751518 B1.

Le document EP1751518 B1 décrit un procédé de mesure du seuil de floculation dans lequel on introduit au moins deux sondes à émetteur et récepteur de lumière dans le milieu à mesurer, ces sondes opérant par transmission optique au niveau de zones de détection de dimensions distinctes. On détermine ensuite laquelle des deux sondes est appropriée à la mesure par détermination du seuil de transmission du milieu avant addition de solvant aliphatique. On détermine enfin à l'aide de la sonde ainsi désignée la floculation après addition de la quantité de solvant aliphatique nécessaire à la floculation. En particulier, l'une des sondes fonctionne en transmission indirecte par réflexion. Le procédé et le dispositif décrits permettent de choisir parmi plusieurs sondes introduites dans un même milieu la sonde la plus adaptée à la mesure, en particulier après ajout de solvant aliphatique. Ainsi, le dispositif peut passer d'une sonde à l'autre après ajout de solvant. Ces changements de sonde, qui correspondent à des changements du chemin optique parcouru par le faisceau lumineux entre un émetteur et son récepteur, sont simples et rapides mais peuvent provoquer des phénomènes d'oscillations du signal susceptibles d'induire des erreurs de détermination du seuil de floculation. La société ROFA® commercialise une sonde présentant un chemin optique de longueur réglable (sonde SVA-130®) qui pourrait permettre d'éviter de telles oscillations. Toutefois, la modification de la longueur du chemin optique nécessite l'intervention d'un opérateur ce qui allonge considérablement le temps de mesure.

Les méthodes aujourd'hui proposées pour la mesure du seuil de floculation des asphaltènes dans des produits hydrocarbonés, présentent donc un certain nombre d'inconvénients. Elles n'offrent pas nécessairement la simplicité, la rapidité et la précision requises des résultats, notamment pour un pilotage en continu d'une unité de traitement, par exemple de viscoréduction et/ou d'une unité de mélanges efficace. Elles ne permettent pas, non plus, l'analyse directe d'une large gamme de produits selon leur teneur en asphaltènes. Elles utilisent des techniques qui ne sont pas facilement automatisables et/ou qui ne sont pas d'une grande simplicité d'utilisation.

La présente invention vise à remédier à un ou plusieurs des inconvénients mentionnés ci-dessus.

RESUME DE L'INVENTION

L'invention a pour objet un dispositif de mesure du seuil de floculation d'un milieu colloïdal par addition de solvant aliphatique, comprenant :

- au moins une cellule de mesure fonctionnant par transmission optique directe et présentant une chambre de mesure définie par des parois fixes, destinée à recevoir le milieu à l'intérieur de la chambre de mesure, et, associé à chaque cellule de mesure :

  - un émetteur de lumière émettant un faisceau lumineux en forme de cône entrant dans la chambre de mesure suivant une direction d'émission,
  - un récepteur de lumière recevant directement le faisceau lumineux sortant de la chambre de mesure,
  - un organe de déplacement motorisé d'un élément choisi parmi l'émetteur, la cellule de mesure et un élément optique situé entre l'émetteur et la cellule de mesure, suivant une direction parallèle à la direction d'émission,

- un système de gestion agencé pour commander l'organe de déplacement motorisé de chaque cellule de mesure pour régler le volume de chaque chambre de mesure traversé par le faisceau lumineux et agencé pour moduler l'intensité lumineuse du faisceau lumineux émis par l'émetteur en faisant varier l'intensité d'un courant continu alimentant l'émetteur de manière à obtenir un signal détectable par le récepteur.

Le système de gestion permet ainsi de modifier le volume de la chambre de mesure traversé par le faisceau lumineux, autrement dit le chemin optique parcouru, et ce pour une unique cellule de mesure associée à un émetteur et un récepteur, le volume de la chambre de mesure en lui-même restant fixe. Ainsi, une seule sonde est nécessaire pour mesurer des produits plus ou moins absorbants, ce qui permet d'éviter les oscillations observées avec le dispositif décrit dans le document EP1751518 B1. Le système de gestion peut notamment permettre de régler le volume éclairé d'une chambre de mesure entre une valeur minimale correspondant à une fraction prédéfinie du volume de la chambre de mesure et une valeur maximale correspondant à la totalité du volume de la chambre de mesure ou à une fraction du volume de la chambre de mesure supérieure à la fraction précédemment mentionnée.

En outre, la cellule de mesure fonctionne par transmission optique directe, autrement dit le faisceau lumineux émis par l'émetteur est reçu directement par le récepteur, sans dispositif optique intermédiaire de réflexion.

L'utilisation d'un organe de déplacement motorisé et d'un système de gestion permet une automatisation du fonctionnement du dispositif et ainsi de s'affranchir d'un opérateur.

Selon l'invention, le système de gestion est agencé pour moduler l'intensité lumineuse du faisceau lumineux émis par l'émetteur, autrement dit la quantité de lumière émise dans la direction d'émission. Plus précisément, par « intensité lumineuse », on entend l'intensité énergétique, à savoir une grandeur radiométrique qui est la mesure de la puissance (ou flux énergétique) d'un rayonnement électromagnétique émis par une source quasiponctuelle, par unité d'angle solide, dans une direction donnée. Son unité dans le système international est le watt par stéradian ($W\ sr^{-1}$). Notamment, la modulation de l'intensité lumineuse mentionnée plus haut est la variation de l'intensité énergétique (la puissance rayonnée dans le cône d'émission) obtenue en faisant varier le courant électrique traversant l'émetteur. Lorsque l'émetteur est une diode électroluminescente, l'intensité énergétique est quasiment proportionnelle au courant électrique traversant la diode.

En outre, la chambre de mesure de chaque cellule de mesure présentant des dimensions fixes, celles-ci peuvent être choisies de sorte que le faisceau lumineux traverse une quantité de matière prédéterminée, par exemple supérieure à celle des sondes existantes, améliorant ainsi la précision de l'appareil même lorsque le volume traversé par le faisceau lumineux est à une valeur minimale.

On notera que le système de gestion peut être relié à d'autres éléments du dispositif et agencé pour les commander/contrôler, tels que le récepteur (pour enregistrer les signaux émis), des capteurs de température, un organe de régulation de température du milieu, ou encore à des électrovannes, voire à des organes de mise en circulation de fluides, pour contrôler la distribution de fluides et éventuellement leur circulation, notamment lorsque le dispositif comprend un circuit tel que décrit plus bas.

Avantageusement, chaque chambre de mesure peut présenter deux éléments optiques fixes formant des parois opposées, l'émetteur et le détecteur associés étant situés en dehors de la chambre de mesure, notamment chacun en regard d'un élément optique selon la direction d'émission.

Par élément optique, on entend un élément apte à être traversé par un faisceau lumineux, notamment sans l'absorber.

Dans certains modes de réalisation, un élément optique de convergence pourra être prévu entre la chambre de mesure et le détecteur afin de faire converger le faisceau lumineux sortant de la chambre de mesure sur le détecteur. Cet élément optique de convergence est donc situé en dehors de la chambre de mesure.

En particulier, chacun des éléments optiques peut être choisi parmi une lame à faces parallèles, une lentille sphérique et une lentille asphérique. De préférence, on pourra choisir une lame à faces parallèles et une lentille asphérique.

Notamment, lorsque la chambre de mesure présente deux lames à faces parallèles, un élément optique de convergence peut être avantageusement disposé entre la chambre de mesure de la cellule de mesure et le récepteur afin de faire converger le faisceau lumineux sortant de la chambre sur le récepteur. Cet élément optique de convergence comprend par exemple une lentille.

Selon un mode de réalisation, chaque organe de déplacement motorisé déplace l'émetteur associé, celui-ci émettant le faisceau lumineux directement sur la chambre de mesure.

Avantageusement, le dispositif de mesure peut comprendre au moins un capteur de température et au moins un organe de régulation de température reliés au système de gestion et le système de gestion peut être agencé pour réguler la température du milieu.

Avantageusement, chaque cellule de mesure peut comprendre une entrée et une sortie de fluide reliant la chambre de mesure à un circuit de fluide associé équipé d'un organe de mise en circulation du fluide. Autrement dit, chaque chambre de mesure d'une cellule de mesure fait alors partie d'un circuit de fluide propre du dispositif de mesure selon l'invention, qui n'est pas en communication avec d'autre(s) circuit(s) de fluide.

Un tel circuit de fluide peut être formé d'une ou plusieurs conduites raccordées les unes aux autres.

En particulier, chaque circuit de fluide peut comprendre un ou plusieurs des éléments suivants :

- au moins un réservoir et au moins une conduite d'injection de liquide reliée à chaque réservoir, optionnellement connectée au circuit par une vanne, notamment une électrovanne,
- une enceinte de mélange présentant une entrée et une sortie connectées au circuit de fluide,
- au moins un organe de régulation de température.

Cet organe de régulation de température peut être choisi parmi un échangeur de chaleur, une résistance de chauffage, un dispositif à effet Peltier ou autre.

Avantageusement, le circuit de fluide peut former une boucle fermée à l'intérieur de laquelle circule le milieu.

Le dispositif de mesure selon l'invention permet de réaliser des mesures en un temps très court, rendant possible des mesures en continu. Ce temps de mesure peut être de l'ordre de la milliseconde, par exemple de 0,5ms.

Aussi, avantageusement, le dispositif peut comprendre des moyens d'injection de liquide en continu, et notamment à débit constant, à l'intérieur du circuit de fluide, notamment à l'intérieur de conduites du circuit de fluide. Ceci permet une injection en continu du solvant aliphatique à l'intérieur du circuit. Une telle injection en continu alors que le liquide circule à l'intérieur du circuit permet d'obtenir rapidement une homogénéisation du mélange. En raison du temps très court de mesure, on peut alors réaliser une mesure au moyen de la cellule de mesure alors que le liquide circule à l'intérieur du circuit de fluide, sans cesser d'ajouter le solvant, ce dernier étant injecté avec un débit constant faible. Cette homogénéisation sera d'autant plus rapide que le solvant sera injecté à l'intérieur de conduites du circuit. Ces moyens d'injection peuvent comprendre une conduite d'injection, une pompe et une électrovanne.

Le dispositif selon l'invention pourra présenter deux ou trois cellules de mesure identiques, chacune associée à un émetteur, un récepteur et un organe de déplacement motorisé, chaque cellule étant reliée à son propre circuit de fluide. Les organes de déplacement motorisé des cellules de mesure pourront être commandés par un même système de gestion.

L'invention a encore pour objet un procédé de mesure du seuil de floculation d'un milieu colloïdal par addition de solvant aliphatique utilisant le dispositif selon l'invention, comprenant l'étape de détermination, à l'aide de la cellule de mesure dudit dispositif, du seuil de floculation après addition de la quantité de solvant aliphatique nécessaire à la floculation.

L'invention fournit aussi un procédé de mesure du seuil de floculation d'un milieu colloïdal par addition de solvant aliphatique, notamment paraffinique, mis en œuvre par le dispositif selon l'invention. Le procédé comprend les étapes suivantes :

(i) on introduit le milieu à l'intérieur de la chambre de mesure définie par des parois fixes de la cellule de mesure fonctionnant par transmission optique directe,

(i1) optionnellement, une étape de dilution dudit milieu avec une quantité prédéterminée de solvant aliphatique préalablement à l'étape (i),

(ii) on règle automatiquement le volume de la chambre de mesure traversé par le faisceau lumineux, et une intensité lumineuse de l'émetteur en faisant varier l'intensité d'un courant continu alimentant l'émetteur, à l'aide du système de gestion de manière à obtenir un signal détectable par le récepteur,

(iii) on détermine le seuil de floculation à l'aide du dispositif de mesure la floculation après addition de la quantité de solvant aliphatique nécessaire à la floculation, optionnellement, le solvant aliphatique est ajouté en continu, et notamment à débit constant, et on procède aux mesures à l'aide du dispositif de mesure alors que le solvant aliphatique est en cours d'addition.

Le dispositif de mesure du seuil de floculation selon l'invention permet de réaliser des mesures dans un temps suffisamment court pour permettre la prise de mesures alors que le solvant aliphatique est en cours d'addition. En particulier, le solvant aliphatique peut alors être injecté à l'intérieur d'un circuit de fluide, notamment à l'intérieur de conduites de ce dernier, le circuit de fluide étant relié à la chambre de mesure de la cellule de mesure, ce circuit de fluide étant équipé d'un organe de mise en circulation du fluide.

Avantageusement, les sondes peuvent être des sondes émettant dans le domaine du NIR et on détermine l'occurrence de la floculation par détermination du pic d'absorption.

Avantageusement, le procédé peut être mis en œuvre à une température prédéterminée réglable, par exemple au moyen d'un organe de régulation de la température. Ceci peut permettre de chauffer le produit, par exemple pour faciliter sa dissolution, par exemple avant addition du solvant aliphatique, mais de réaliser la mesure à une température prédéterminée plus basse.

Selon un mode de réalisation, le milieu colloïdal comprend des asphaltènes.

L'invention fournit encore un procédé de détermination de la stabilité d'un mélange comprenant des asphaltènes par mise en œuvre au moins deux fois du procédé de mesure du seuil de floculation d'un milieu colloïdal selon l'invention sur un milieu contenant le mélange et une quantité donnée de solvant aromatique, à des taux de dilution différents. Le procédé de mesure du seuil de floculation peut notamment être mis en œuvre au moins deux fois successivement dans une même cellule de mesure d'un dispositif de mesure ou être mis en œuvre simultanément dans deux ou plusieurs cellules de mesure identiques d'un même dispositif de mesure.

Selon un mode de réalisation, le couple solvant aromatique/solvant aliphatique (notamment paraffinique) utilisé est le couple toluène/n-heptane.

BREVE DESCRIPTION DES FIGURES

La figure 1 est une représentation du graphe aromaticité du solvant en fonction de l'inverse de la dilution, c'est à dire la courbe de précipitation d'un produit noir qui, à un taux de dilution déterminé de ce même produit noir, associe l'aromaticité minimale du solvant nécessaire pour que le mélange ne précipite pas.

La figure 2 est une représentation schématique d'un dispositif selon un mode de réalisation de l'invention.

Les figures 3a et 3b sont des représentations schématiques d'une cellule de mesure selon un mode de réalisation du dispositif de l'invention, dans lesquelles l'émetteur occupe des positions différentes.

La figure 4 est une représentation schématique d'une cellule de mesure selon un autre mode de réalisation du dispositif.

Les figures 5a, 5b et 5c représentent respectivement les valeurs S, Sa et So du produit noir BP3 en fonction du nombre d'essais. Les figures 6a, 6b et 6c représentent respectivement les valeurs S, Sa et So du produit noir BP14 en fonction du nombre d'essais. Toutes ces figures présentent la même légende, représentée uniquement figure 5a.

## EXPOSE DETAILLEE DES MODES DE REALISATION DE L'INVENTION

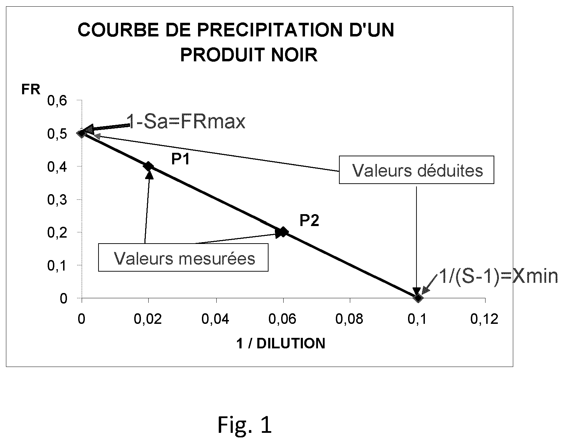

En référence à la figure 1, on décrit la méthode utilisant le dispositif décrit dans le document EP1751518 B1 pour la détermination des valeurs de S, So et Sa, pour un mélange donné de produit noir.

La stabilité intrinsèque de tout système colloïdal est quantifiée par une dilution à l'aide d'un solvant paraffinique d'un produit noir, tel qu'un fioul, un résidu de distillation atmosphérique du pétrole (ou sous vide), un pétrole brut, préalablement mélangé avec un solvant aromatique. Cette stabilité intrinsèque (S) dépend du caractère aromatique des asphaltènes (Sa) et du caractère aromatique du milieu (So), comme il a été décrit plus haut. On détermine ainsi la stabilité intrinsèque S d'un système colloïdal par mesure du seuil de floculation d'au moins 2 mélanges différents. A partir d'au moins ces 2 points, on trace une droite, dite de précipitation d'un produit noir (fig 1), qui permet d'accéder aux paramètres Sa et S, puis par calcul, à la valeur So.

Par ajout d'un solvant paraffinique dans le produit noir, le mélange devient instable à partir d'un certain taux de dilution Xmin, appelé "taux de dilution minimal".

On utilise les définitions suivantes, telles que définies dans la norme ASTM D7157-18 (Révision 2018):

- Taux de dilution X (ml/g):

  volume de solvant total (aromatique + paraffinique) en millilitres/masse de produit noir en grammes.

- stabilité intrinsèque S du produit noir:
  S = 1 + Taux de dilution minimal. On retrouve ici la notion de S-1 comme réserve de stabilité.

Pour les mesures expérimentales, on utilise deux types de solvants, le premier est aromatique, constitué essentiellement de molécules aromatiques pour la dilution de l'échantillon (par exemple toluène, xylène, ou encore 1-méthylnaphtalène) et le second est aliphatique de type solvant paraffinique (par exemple n-heptane, cétane, ou encore iso-octane) pour provoquer la floculation des asphaltènes.

Le taux de floculation FR (« flocculation ratio ») est défini comme suit :
FR=volume de solvant aromatique/volume de solvant total.

L'aptitude des asphaltènes à être peptisés (« peptizability of an asphaltene ») est définie par : Sa=1 - FRmax, où FRmax est le taux de floculation maximal (à 1/X =0).

On appelle courbe de précipitation la fonction du taux de floculation FR en fonction du taux de dilution, soit ici :

$$1\text{-}Sa=f(1/X)=A + B/X.$$

A et B sont des constantes qui dépendent uniquement de l'échantillon et permettent d'accéder aux valeurs de S, So et Sa.

On procède comme suit. On part d'un premier mélange d'une masse donnée de produit noir dans une quantité donnée de solvant aromatique et on ajoute par incréments successifs un solvant paraffinique. On détermine le seuil de floculation (notamment par une méthode utilisant une sonde IR) et on note alors le taux de dilution et le taux de floculation FR associé au mélange analysé. On obtient un premier point, identifié par le point P1 sur le graphe (fig 1). On recommence l'opération, avec un produit de départ qui est initialement moins fortement dilué dans le solvant aromatique. On obtient alors une autre mesure matérialisée par le point P2. Avec les deux points P1 et P2 il est alors possible de tracer la droite passant par ces points et d'obtenir des valeurs limites (1-Sa) sur l'axe des ordonnées (FRmax ou taux de dilution infini) et 1/(S-1) sur l'axe des abscisses (FR nul). Il devient ensuite possible d'accéder aux valeurs de S, Sa puis So par le calcul.

Cette technique, qui fait référence à la méthode normalisée ASTM D7157-18 (Révision 2018), et qui consiste en la construction d'une courbe de précipitation, à partir d'au moins deux résultats de mesures (trois dans la norme), pour ensuite déterminer les valeurs des aromaticités limites et nulles, est celle généralement suivie dans l'invention. Les masses, les volumes et les produits utilisés sont tout à fait classiques dans l'art de ce type d'analyse.

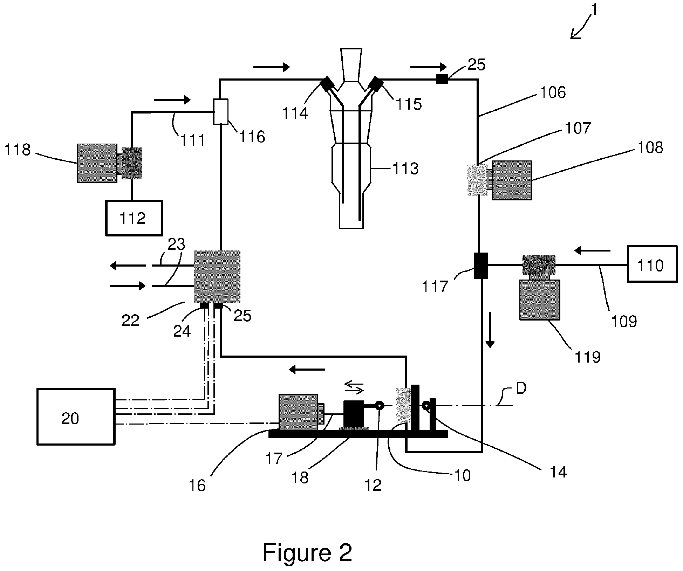

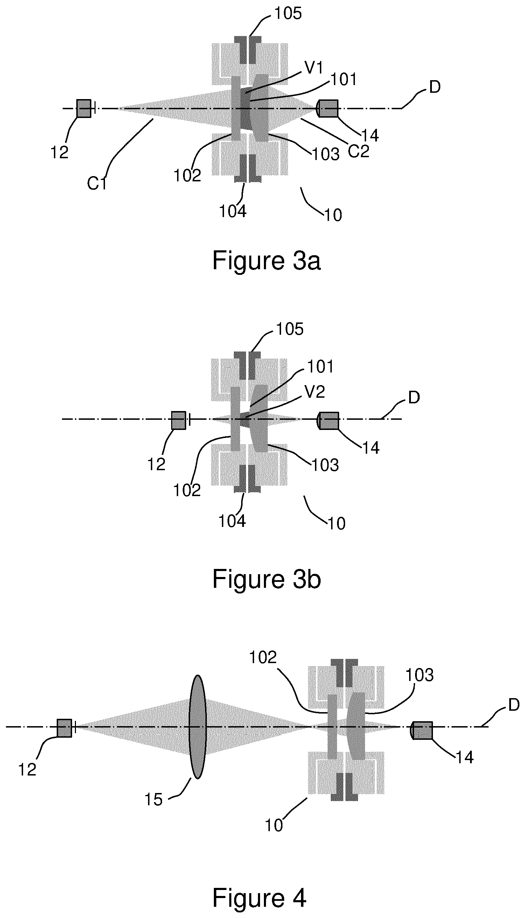

En référence à la figure 2 et aux figures 3a, 3b, 4, le dispositif (1) selon l'invention comprend une cellule de mesure (10) fonctionnant par transmission optique directe présentant une chambre de mesure (101) de dimensions fixes, définie par des parois fixes.

Le dispositif (1) comprend également, associés à la cellule de mesure (10), un émetteur de lumière (12) émettant (configuré pour émettre) un faisceau lumineux entrant dans la chambre de mesure (101) suivant une direction d'émission (D) et un récepteur de lumière (14) recevant directement le faisceau lumineux sortant de la chambre de mesure (101). Autrement dit, le récepteur de lumière (14) est positionné de manière à recevoir directement le faisceau lumineux sortant. Il peut notamment être positionné dans la direction d'émission (D), d'un côté de la chambre de mesure (101) opposé au côté où est situé l'émetteur de lumière (12), tel que représenté sur les figures. L'émetteur est un émetteur IR classique, par exemple une diode électroluminescente, de même que le récepteur, ce dernier étant un récepteur photoélectrique apte à délivrer un courant lorsqu'il reçoit un flux lumineux. Le diamètre utile de l'émetteur est notamment de 4,0-5,0, par exemple de 4,7-4,8 mm.

Le dispositif (1) comprend également un organe de déplacement motorisé (16) d'un élément, ici l'émetteur (12), suivant une direction parallèle à la direction d'émission (D) de l'émetteur. L'organe de déplacement motorisé, configuré pour déplacer un élément, est par exemple un moteur électrique, notamment un moteur pas à pas. L'axe (17) de ce moteur peut être relié à l'émetteur (12) afin de le déplacer en translation, l'émetteur (12) étant par exemple supporté sur un socle mobile (18), par exemple monté sur des rails (non représentés).

Dans le mode de réalisation représenté, le dispositif (1) comprend en outre un système de gestion (20) au moins de l'organe de déplacement (16) motorisé permettant l'automatisation du réglage du volume de la chambre de mesure traversé par le faisceau lumineux. Il ne s'agit donc pas de modifier le volume de la chambre de mesure, qui est fixe, mais uniquement le volume de la portion de la chambre de mesure éclairée par le faisceau lumineux. Dans l'exemple, le système de gestion est également agencé pour contrôler l'intensité lumineuse émise par l'émetteur (12).

Ce système de gestion (20) peut comprendre un ou plusieurs processeurs de type microprocesseur, microcontrôleur ou autre, par exemple faisant partie d'un ordinateur. Le ou les processeurs comportent notamment des moyens d'exécution de programme d'ordinateur adaptés pour mettre en œuvre le procédé décrit dans la présente invention.

Dans un mode de réalisation, le système de gestion peut être agencé pour recevoir des données. Le système de gestion peut également être agencé pour transmettre des données, notamment vers un dispositif d'affichage tel qu'un écran. Le système de gestion peut ainsi comprendre une ou plusieurs interfaces d'entrée, de sortie, ou d'entrée/sortie. Il peut s'agir d'interfaces de communication sans fil (Bluetooth, WIFI ou autre) ou de connecteurs (port réseau, port USB, port série, port Firewire®, port SCSI ou autre).

Dans un mode de réalisation, le système de gestion peut comporter des moyens de mémorisation qui peuvent être une mémoire vive ou une mémoire RAM (de l'anglais « Random Access Memory »), une EEPROM (de l'anglais « (Electrically-Erasable Programmable Read-Only Memory »), une mémoire flash, une mémoire externe, ou autre. Ces moyens de mémorisation peuvent notamment mémoriser les données reçues, et éventuellement de(s) programme(s) d'ordinateur.

Dans le mode de réalisation représenté, contrairement aux sondes usuelles existantes, la chambre de mesure (101) fait partie de la cellule de mesure (10) mais n'est pas définie par l'émetteur (12) et le récepteur (14) bien qu'elle soit située entre ces derniers permettant au faisceau lumineux de traverser la chambre de mesure. La chambre de mesure (101) est ici définie en partie par deux éléments optiques (102, 103) fixes qui forment des parois opposées de la chambre de mesure. Autrement dit, l'émetteur et le récepteur sont situés en dehors de la chambre de mesure et sont distincts de celle-ci.

Un premier élément optique (102) situé du côté de l'émetteur (12), ici une lame à faces parallèles, permet la transmission du faisceau lumineux provenant de l'émetteur (12) à l'échantillon situé à l'intérieur de la chambre de mesure (101). Un deuxième élément optique (103) situé du côté du détecteur (14), ici une lentille asphérique, permet de focaliser le faisceau lumineux transmis par l'échantillon sur le détecteur (14).

D'autres couples d'éléments optiques précédemment listés peuvent être envisagés, toutefois, la configuration de l'exemple présente l'avantage d'être particulièrement performante. Ces différents éléments optiques peuvent être réalisés en verre, polymère, métalloïde, mais également en matière hybride (verre/polymère).

Dans l'exemple, l'émetteur (12) est mobile en translation et déplacé au moyen de l'organe de déplacement motorisé (16). La cellule de mesure (10) et le détecteur (14) sont fixes. L'émetteur (12) peut notamment être déplacé entre :

- une première position (fig. 3a) dans laquelle le cône d'émission (C1) du faisceau lumineux est maximal de sorte que la totalité, ou la quasi-totalité, du volume (V1) de la chambre de mesure (101) est traversé par le faisceau lumineux, et
- une deuxième position (fig.3b) dans laquelle le cône d'émission (C1) du faisceau lumineux présente un angle au sommet plus faible de sorte que seule une portion (V2) du volume de la chambre de mesure (101) est traversée par le faisceau lumineux.

On comprend ainsi que le déplacement de l'émetteur permet de régler le volume de la chambre de mesure (101) traversé par le faisceau lumineux.

Dans les deux positions, on notera que le cône de transmission du faisceau lumineux C2 converge sur le détecteur (14) fixe.

En variante, l'organe de déplacement motorisé (16) pourrait déplacer la cellule de mesure (10), l'émetteur et le détecteur étant fixes, ou encore, tel que représenté figure 4 un élément optique (15), par exemple une lentille, situé entre l'émetteur (12) fixe et la cellule de mesure (10) fixe. Dans ce cas, on peut faire varier le demi-angle au sommet du cône de lumière arrivant sur la chambre de mesure en rapprochant ou en éloignant la lentille de la cellule de mesure (10). On comprend ainsi que l'élément optique (15) est optionnel.

Dans le mode de réalisation représenté, le dispositif (1) comprend en outre deux organes de régulation de température (22), ici un échangeur de chaleur à l'intérieur duquel on peut faire circuler par exemple un liquide réfrigérant (23), et un organe de chauffage (24). Cet organe de chauffage pourrait également être situé autour de l'enceinte de mélange (113) décrite plus bas, tel qu'un bloc thermostaté ou autre. Il comprend également un ou plusieurs capteurs de température (25), par exemple un capteur de température situé au niveau de l'échangeur de chaleur et un capteur de température au niveau de la cellule de mesure, à l'entrée ou à la sortie de celle-ci, ou dans le circuit de fluide tel que représenté. L'invention n'est toutefois pas limitée par une position particulière des capteurs de température. On pourra notamment en positionner un en amont de la chambre de mesure par rapport à la circulation du fluide.

Ces éléments peuvent être contrôlés par le système de gestion (20) lequel peut alors être agencé pour une gestion automatique de la température du milieu.

La cellule de mesure (10) pourrait être plongée dans le milieu de manière à ce que ce dernier remplisse entièrement la chambre de mesure. Toutefois, de préférence, tel que représenté sur les figures 2 à 4, la cellule de mesure (10) comprend une entrée (104)de fluide et une sortie (105) de fluide reliant la chambre de mesure (101) à un circuit de fluide (106), lequel est équipé d'un organe de mise en circulation du fluide (107), ici une pompe péristaltique (107) commandée par un moteur pas à pas (108). La chambre de mesure peut ainsi se présenter sous la forme d'une simple conduite ouverte à ses deux extrémités, à section transversale fermée.

Plus particulièrement, dans l'exemple, le circuit de fluide (106) comprend :

- une première conduite d'injection (109) de liquide reliée à un réservoir (110) pour l'injection d'un premier solvant, par exemple le solvant aromatique,
- une deuxième conduite d'injection (111) de liquide reliée à un deuxième réservoir (112) pour l'injection d'un deuxième solvant, par exemple le solvant paraffinique,
- une enceinte de mélange (113) présentant une entrée (114) et une sortie (115) connectées au circuit de fluide (106), pour recevoir le milieu,
- l'organe de régulation de température (22) et l'organe de chauffage (24) précédemment mentionnés.

Les conduites d'injection (109) et (111) peuvent être équipées d'électrovannes (116), (117), et d'une pompe (118, (119) lesquelles sont de préférence commandées par le système de gestion (20) pour une automatisation du dispositif.

Le circuit de fluide (106) forme ici une boucle qui peut donc être fermée pour la circulation du milieu à l'intérieur de la boucle par exemple dans le sens de circulation symbolisé par les flèches sur la figure 2.

On pourra éventuellement prévoir un système de chauffage de l'enceinte de mélange et une colonne de reflux pour permettre de chauffer à reflux le produit contenu dans l'enceinte afin de faciliter la dissolution de l'échantillon.

Le fonctionnement du dispositif selon l'invention est décrit ci-après.

On introduit l'échantillon à analyser à l'intérieur de la chambre de mesure de la cellule de mesure du dispositif selon l'invention. Dans le dispositif représenté, on introduit l'échantillon dans l'enceinte de mélange avant de le faire circuler dans le circuit et à l'intérieur de la chambre de mesure. Notamment, le volume de produit est suffisant pour remplir entièrement au moins la chambre de mesure.

Dans l'exemple, cette étape d'introduction est suivie d'une étape d'ajout du solvant aromatique au produit pour former le milieu à analyser. L'échantillon est alors dilué par le solvant aromatique avant de circuler à l'intérieur de la chambre de mesure de la cellule de mesure.

On procède ensuite à une étape de réglage au cours de laquelle on règle le volume de la chambre de mesure de la cellule de mesure traversée par le faisceau lumineux, avant l'ajout de solvant paraffinique, c'est-à-dire avant la floculation. On pourra avantageusement également régler l'intensité lumineuse émise par l'émetteur. Cette étape de réglage permet d'obtenir un signal détectable par le récepteur. Lors de cette étape, on peut procéder de préférence d'abord au réglage de l'intensité lumineuse émise par l'émetteur puis au réglage du volume afin d'obtenir un signal détectable. Par signal détectable, on entend un signal pouvant être distingué d'un bruit de fond et qui n'est pas saturé.

On détermine enfin le seuil de floculation à l'aide du dispositif de mesure de la floculation après addition de la quantité de solvant paraffinique nécessaire à la floculation. A cet effet, on ajoute progressivement le solvant paraffinique et on note la chute de transmission correspondant à la floculation des asphaltènes. Cette détermination se fait par des techniques classiques, par exemple, par mesure du pic d'absorption.

Le réglage du volume de la chambre de mesure traversé par le faisceau lumineux est réalisé automatiquement, par un programme informatique prédéterminé à la construction du dispositif. Ce réglage automatique peut inclure un réglage de l'intensité lumineuse émise par l'émetteur.

Il peut en effet être préférable de moduler l'intensité lumineuse émise par l'émetteur afin d'atteindre une valeur de consigne correspondant à une valeur minimale mesurable par le détecteur. Ce réglage de l'intensité lumineuse peut être obtenu en faisant varier l'intensité d'un courant continu alimentant l'émetteur.

De manière connue, un détecteur peut détecter un faisceau lumineux dans une plage de détection déterminée, correspondant à un pourcentage de la lumière émise par l'émetteur : en dessous de la valeur minimale de cette plage, aucun signal n'est détecté, au dessus de la valeur maximale de la plage la saturation du récepteur entraîne une perte de sensibilité. La valeur de consigne est généralement choisie dans une partie de la plage de détection proche de la valeur minimale.

Le réglage est par exemple réalisé de la manière suivante. L'intensité lumineuse émise par l'émetteur est tout d'abord réglée à sa valeur minimale, correspondant par exemple à un courant de 6mA, le volume de la chambre de mesure traversé par le faisceau lumineux étant à une valeur maximale, par exemple de l'ordre de 500µl. On augmente alors l'intensité lumineuse émise par l'émetteur jusqu'à atteindre la valeur de consigne ou une valeur maximale de l'intensité lumineuse émise, correspondant par exemple à un courant de 100mA. Lorsque la valeur de consigne ne peut être atteinte en augmentant l'intensité lumineuse émise jusqu'à cette valeur maximale, le volume de la chambre de mesure est réduit progressivement jusqu'à ce que la valeur de consigne soit atteinte ou jusqu'à une valeur minimale du volume, par exemple de l'ordre de 10µl. On effectuera alors les mesures dans ces conditions. En particulier, l'intensité lumineuse émise par l'émetteur et le volume de la chambre de mesure restent fixes au fur et à mesure de la dilution par le solvant paraffinique. De cette façon, le signal peut être mesuré avec une bonne précision avec une seule et unique cellule de mesure réglée de manière appropriée, ce qui offre un gain de temps appréciable pour l'opérateur.

La valeur minimale de l'intensité lumineuse émise par l'émetteur correspond par exemple à une valeur en dessous de laquelle la précision de la mesure est trop faible pour permettre de distinguer un signal du bruit de fond. Cette valeur minimale correspond par exemple à un courant de 6mA.

La valeur minimale du volume de la chambre de mesure correspond par exemple à 10µl. Elle peut être déterminée expérimentalement par des mesures avec des échantillons très opaques. Ce volume minimal pourrait être augmenté afin de permettre/d'améliorer la détection de floculation de milieux contenant de très faibles quantités d'asphaltènes.

Selon un mode de réalisation avantageux, mettant notamment en œuvre le dispositif décrit en référence aux figures, l'étape d'introduction comprend une phase de dissolution, au cours de laquelle on introduit le milieu à l'intérieur de l'enceinte de mélange (113), en une quantité suffisante pour remplir entièrement le circuit (106), puis on régule la température du milieu à une température de dissolution au moyen de l'organe de régulation de température (22) et de l'organe de chauffage (24), ou au moyen d'un organe de régulation de température entourant l'enceinte de mélange (113). Le milieu contenu dans l'enceinte de mélange (113) pourra également être maintenu sous agitation. On injecte ensuite le solvant aromatique à l'intérieur du circuit et l'on met en circulation le milieu et le solvant aromatique dans le circuit (106) au moyen de la pompe (107) pendant suffisamment de temps pour obtenir un mélange homogène.

Cette phase de dissolution peut éventuellement être suivie d'une phase de pré-dilution avec le solvant paraffinique, au cours de laquelle une quantité prédéterminée de ce solvant peut être injectée dans le circuit. On opère de cette manière dans le cas de produit très aromatique et stable ou lorsque le produit est trop sombre et l'intensité lumineuse émise par détecteur atteint son maximum sans avoir détecté le volume de floculation.

On réalise ensuite une phase de refroidissement au cours de laquelle on régule la température à une température d'essai prédéterminée au moyen de l'organe de de régulation de température (22).

On procède ensuite à une phase de dosage au cours de laquelle on ajoute progressivement le solvant paraffinique. Cette addition de solvant peut être réalisée par des additions incrémentales ou par une addition en continue. On procède alors à l'acquisition et à l'enregistrement du signal du détecteur soit après chaque ajout de solvant, soit en cours d'addition du solvant. Dans ce dernier cas, le débit d'introduction du solvant à l'intérieur du circuit pourra être constant, par exemple de l'ordre de 1mL/minute. On notera que le produit à analyser circule dans le circuit en cours d'addition du solvant et d'acquisition du signal. Cette phase de dosage peut être arrêtée par un opérateur, lorsque le volume maximal de la cellule de mélange est atteint ou lorsqu'un nombre d'additions incrémentales prédéterminé a été effectué ou lorsqu'un volume de solvant prédéterminé a été ajouté.

On peut ensuite procéder à une phase de nettoyage en faisant par exemple circuler le solvant aromatique dans le circuit.

L'invention est décrite en référence à un dispositif comprenant une unique cellule de mesure. On notera toutefois que le dispositif de l'invention peut comprendre plusieurs cellules de mesure indépendantes identiques, par exemple trois, afin de réaliser simultanément en parallèle trois essais sur un produit.

En outre, le dispositif selon l'invention permet d'obtenir un domaine d'application spectral possible pour les mesures qui est très large. Le dispositif selon l'invention est approprié pour la détermination des valeurs de S, Sa et So pour tous types de résidus et fuels et n'est pratiquement pas limité quant à la nature du milieu à tester. Comme le dispositif comprend un seul type de cellule de mesure, il est possible de procéder à plusieurs mesures pour mesurer un même produit en un temps moindre. On peut ainsi effectuer 3 mesures et donc obtenir 3 points de la courbe et par-là une bonne répétabilité des mesures pour S, Sa et So. Enfin, la méthode de détermination selon l'invention peut être mise en œuvre à température ambiante ou à une température prédéterminée, ce qui permet de mesurer les paramètres S, Sa et So à une température donnée et de vérifier leur évolution en fonction de la température, puisque la stabilité des asphaltènes dépend de la température.

En général, le couple solvant aromatique/solvant paraffinique utilisé dans l'invention est le couple toluène/n-heptane.

EXEMPLES

Les exemples suivants illustrent l'invention sans la limiter. L'invention est définie par les revendications.

Exemple 1

Des mesures ont été effectuées sur 13 échantillons de produits noirs différents pour lesquels les valeurs S, Sa ont été mesurées et So calculées, d'une part avec une méthode utilisant les sondes SVA-130® proposées par la société ROFA mettant en œuvre la méthode décrite dans la norme ASTM D7157-18 (Révision 2018) ("Méthode de mesure A" dans le tableau 1 ci-dessous) et d'autre part avec le dispositif et le procédé conformes à la présente invention ("Méthode de mesure B").

Le dispositif conforme à la présente invention est du type décrit en références aux figures 2 et 3. La cellule de mesure comprend notamment une lame à faces parallèles et une lentille asphérique, la distance entre la fenêtre et la lentille étant de 0.5 mm au centre et de 3 mm sur les bords.

Le volume de la boucle de circuit est ici de 4ml. Les mesures sont réalisées alors que le fluide circule à une vitesse de 10mL/min environ. La température d'essai est ici la température ambiante. Il est possible de chauffer le mélange solvant aromatique/produit pour accélérer la dissolution de ce dernier, notamment dans les cas des résidus sous vide. Un chauffage de 60°C à 100°C suffit à dissoudre le produit dans ce cas en quelques minutes. Dans certains cas (produits très stables), une pré-dilution au n-heptane a été réalisée avant le début des mesures afin d'éviter une saturation du détecteur.

Dans cet exemple, les produits noirs repérés BP1 à BP13 correspondent à :

- BP1 : Résidu atmosphérique Visco-réduit basse teneur en soufre préfluxé (pré dilué avec un fluxant) ;
- BP2 : Résidu atmosphérique Visco-réduit haute teneur en soufre préfluxé ;
- BP3 : Résidu atmosphérique Visco-réduit haute teneur en soufre préfluxé ;
- BP4 : Résidu sous vide Visco-réduit ;
- BP5, BP7, BP8 : résidu sous vide ;
- BP6 : Résidu sous vide basse teneur en soufre ;
- BP9, BP11 : slurry ;
- BP10, BP12, BP13 : mélanges fiouls très instables.

On note que les valeurs obtenues avec la méthode de mesure B selon l'invention sont proches des valeurs obtenues avec la méthode de mesure A, les sondes SVA-130® permettant une mise en œuvre de la norme ASTM D7157-18 (Révision 2018) dans le respect des conditions de répétabilité et de reproductibilité définies dans cette norme.

Pour chacune des 13 mesures, le coefficient de corrélation $R^2$ de la courbe de précipitation (taux de floculation FR en fonction de l'inverse de la dilution) construite avec 3 points (P1, P2 et P3) varie de 0,9817 à 0,9999, ce qui est supérieur à la valeur de $R^2$ minimale (0,98) exigée par la norme.

De plus, l'automatisation de l'analyse permet de réaliser l'analyse complète en moins d'une heure avec la méthode de mesure B selon l'invention alors qu'il faut plus de deux heures pour la méthode A en particulier en raison du temps opérateur nécessaire pour modifier le trajet optique des sondes SVA-130®. En outre, la méthode de mesure B selon l'invention est également plus rapide qu'en utilisant un dispositif et une méthode conformes au document EP1751518 B1 du fait notamment de l'automatisation de la dilution.

Tableau 1 Comparaison des résultats des mesures S et Sa, et du calcul de So, avec les sondes ROFA SVA-130® (Méthode de mesure A) et le dispositif et procédé conformes à l'invention (Méthode de mesure B)

| Produits | Méthode de mesure A | | | Méthode de mesure B | | |
|---|---|---|---|---|---|---|
| | S | Sa | So | S | Sa | So |
| **BP1** | 1,62 | 0,51 | 0,8 | 1,89 | 0,483 | 0,975 |
| **BP2** | 1,73 | 0,45 | 0,93 | 1,93 | 0,432 | 1,1 |
| **BP3** | 1,93 | 0,62 | 0,74 | 2,01 | 0,612 | 0,779 |
| **BP4** | 1,64 | 0,36 | 1,04 | 1,65 | 0,353 | 1,07 |
| **BP5** | 7,9 | 0,85 | 1,16 | 7,59 | 0,883 | 0,89 |
| **BP6** | 4,27 | 0,86 | 0,6 | 5,01 | 0,86 | 0,701 |
| **BP7** | 4,29 | 0,81 | 0,83 | 4,92 | 0,815 | 0,923 |
| **BP8** | 1,53 | 0,46 | 0,83 | 1,67 | 0,43 | 0,95 |

(suite)

| Produits | Méthode de mesure A | | | Méthode de mesure B | | |
|---|---|---|---|---|---|---|
| | S | Sa | So | S | Sa | So |
| BP9 | 1,38 | 0,14 | 1,18 | 1,43 | 0,172 | 0,19 |
| BP10 | 1,31 | 0,61 | 0,51 | 1,35 | 0,659 | 0,461 |
| BP11 | 1,63 | 0,24 | 1,25 | 1,76 | 0,258 | 1,3 |
| BP12 | 1,69 | 0,6 | 0,68 | 1,73 | 0,615 | 0,666 |
| BP13 | 1,43 | 0,44 | 0,81 | 1,45 | 0,408 | 0,856 |

Exemple 2

Afin de comparer les valeurs des répétabilités obtenues sur les mesures de S, Sa puis du So calculé, accessibles par la méthode utilisant les sondes ROFA SVA-130® (Méthode de mesure A) et celle automatisée dont le dispositif et le procédé font l'objet de la présente invention (Méthode de mesure B), 2 échantillons BP3 et BP14 ont été choisis, l'échantillon BP3 est défini dans l'exemple 1, l'échantillon BP14 est un brut provenant du Koweit, liquide à une température inférieure à 30°C.

Les tableaux 2 et 3 ci-dessous rassemblent les valeurs moyennes calculées pour 11 mesures distinctes pour la méthode de mesure B selon l'invention et des valeurs moyennes calculées sur une dizaine de mesures distinctes pour la méthode de mesure A. Les valeurs de répétabilité et de reproductibilité calculées en utilisant les formules figurant dans la norme ASTM D7157-18 (Révision 2018) à partir de la moyenne calculée pour les mesures de chacune des méthodes de mesure A et B figurent également dans ces tableaux. Dans les tableaux 2 et 3, figurent également l'écart type pour les 11 mesures de la méthode de mesure B, ainsi, que la répétabilité calculée selon la formule générale : 2 x racine carrée de 2 x écart type, soit 2,83 x écart type.

L'efficacité figurant dans ces tableaux est le rapport de la répétabilité ASTM (selon ASTM D7157-18 -Révision 2018) sur la répétabilité calculée avec la formule générale.

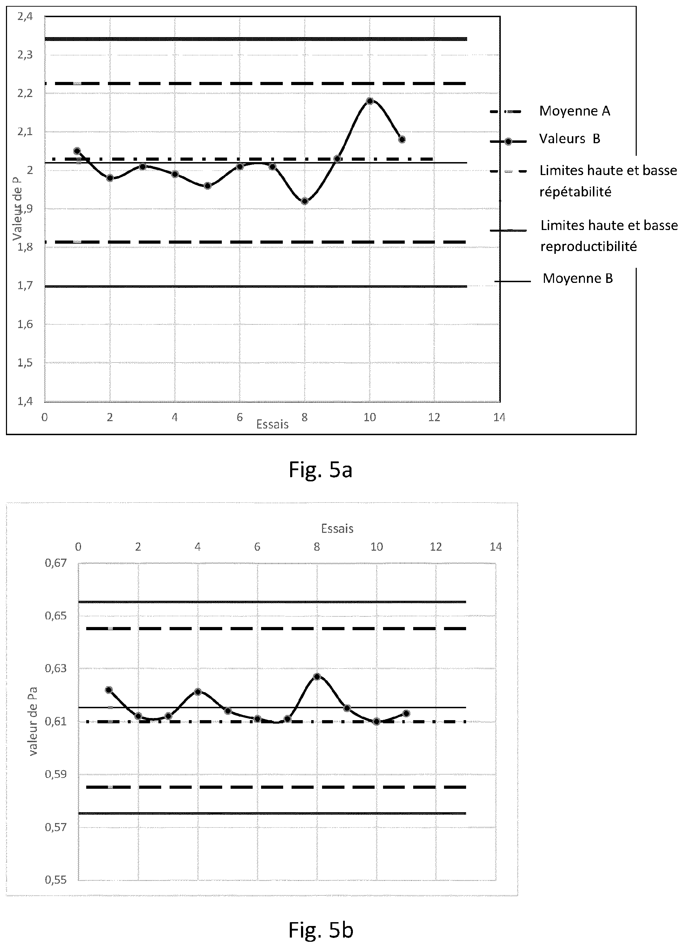

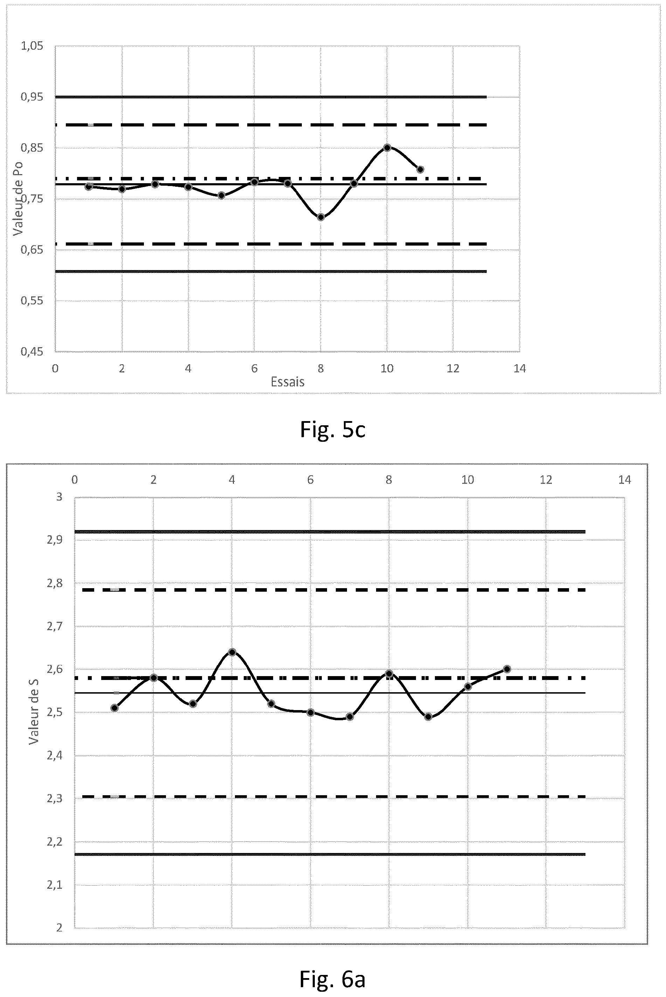

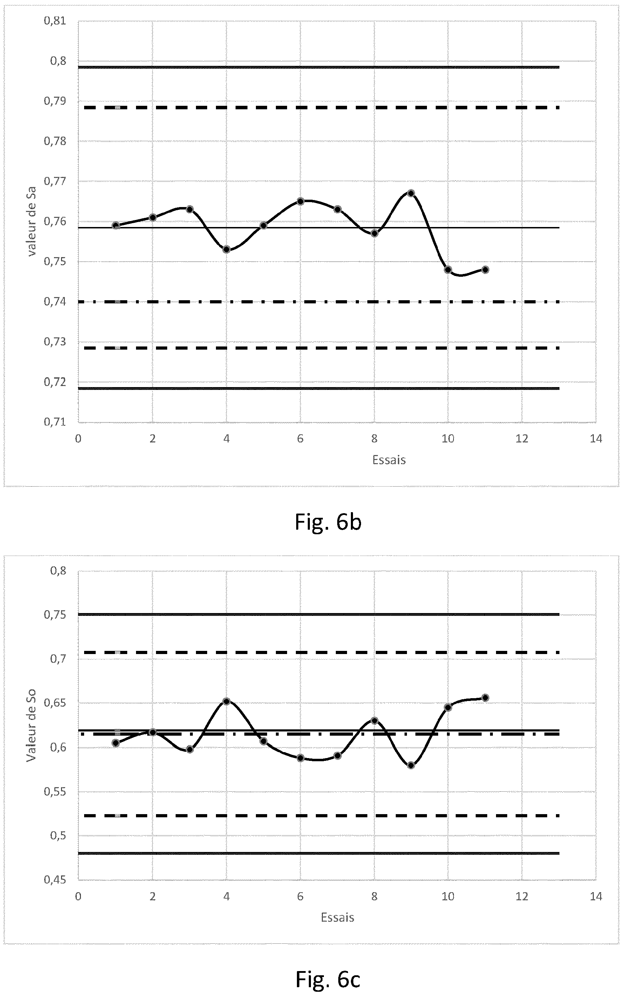

Les figures 5a, 5b et 5c représentent respectivement les valeurs S, Sa et So du produit noir BP3, les figures 6a, 6b et 6c représentent respectivement les valeurs S, Sa et So du produit noir BP14 pour les 11 mesures.

Sur chacune de ces figures, sont représentées :

- des limites haute et basse de S, Sa et So tenant compte de la répétabilité (limites hautes et basses de répétabilité), calculées respectivement en ajoutant et en retranchant la valeur de répétabilité calculée pour la méthode de mesure B à la moyenne des mesures calculée pour la méthode de mesure B,
- des limites haute et basse S, Sa et So tenant compte de la reproductibilité (limites hautes et basses de reproductibilité), calculées respectivement en ajoutant et en retranchant la valeur de reproductibilité calculée pour la méthode de mesure B à la moyenne des mesures calculée pour la méthode de mesure B,
- la moyenne des valeurs obtenues avec la méthode de mesure B selon l'invention (Moyenne B),
- les valeurs obtenues avec la méthode de mesure B selon l'invention (Valeurs B),
- la moyenne des valeurs obtenues avec la méthode de mesure A (Moyenne A).

On note ainsi que les valeurs figurant dans les tableaux 2 et 3 sont relativement proches entre les méthodes de mesure A et B, comme le montrent également les courbes 5a, 5b, 5c relatives à l'échantillon BP3 et les courbes 6a, 6b, 6c relatives à l'échantillon BP14.

Par ailleurs, la notion d'efficacité exprimée dans les tableaux 2 et 3 permet de comparer si la répétabilité de la norme ASTM D7157-18 (Révision 2018) est plus petite ou plus grande que la répétabilité propre au dispositif selon l'invention. Notamment, plus la répétabilité est petite, plus les valeurs sont répétables et donc moins variables. On notera en particulier que la valeur de l'efficacité est toujours supérieure à 1, ce qui signifie que la répétabilité du dispositif selon l'invention est plus petite que la répétabilité de la norme ASTM D7157-18 (Révision 2018).

Sur chaque figure 5a, 5b, 5c, 6a, 6b, 6c, on constate que les minimas et les maximas des courbes des essais effectués selon la méthode de mesure B sont situés entre les limites basse et haute de répétabilité et de reproductibilité. Autrement dit, les écarts de valeurs entre plusieurs mesures obtenus avec la méthode de mesure B selon l'invention sont faibles pour les deux types de produits.

Tableau 2 Mesures des répétabilités sur les valeurs S, Sa et So sur l'échantillon BP3 obtenues avec les sondes ROFA SVA-130® (Méthode de mesure A) et le dispositif et procédé conformes à l'invention (Méthode de mesure B)

| | BP3 | | | | | |
|---|---|---|---|---|---|---|
| | Méthode de mesure A | | | Méthode de mesure B | | |
| | S | Sa | So | S | Sa | So |
| Moyenne | 1,930 | 0,620 | 0,740 | 2,020 | 0,615 | 0,779 |
| Reproductibilité ASTM | 0,313 | 0,040 | 0,163 | 0,322 | 0,040 | 0,171 |
| Répétabilité ASTM | 0,200 | 0,030 | 0,111 | 0,206 | 0,030 | 0,117 |
| Ecart type | - | - | - | 0,07 | 0,01 | 0,03 |
| Répétabilité | - | - | - | 0,193 | 0,016 | 0,093 |
| Efficacité | - | - | - | 1,07 | 1,90 | 1,26 |

Tableau 3 Mesures des répétabilités sur les valeurs S, Sa et So sur l'échantillon BP14 obtenues avec les sondes ROFA SVA-130® (Méthode de mesure A) et le dispositif et procédé conformes à l'invention (Méthode de mesure B)

| | BP14 | | | | | |
|---|---|---|---|---|---|---|
| | Méthode de mesure A | | | Méthode de mesure B | | |
| | S | Sa | So | S | Sa | So |
| Moyenne | 2,580 | 0,740 | 0,660 | 2,548 | 0,757 | 0,619 |
| Reproductibilité ASTM | 0,378 | 0,040 | 0,145 | 0,375 | 0,040 | 0,136 |
| Répétabilité ASTM | 0,242 | 0,030 | 0,099 | 0,240 | 0,030 | 0,093 |
| Ecart type | - | - | - | 0,05 | 0,006 | 0,027 |
| Répétabilité | - | - | - | 0,14 | 0,018 | 0,076 |
| Efficacité | - | - | - | 1,66 | 1,65 | 1,22 |

Exemple 3

Le dispositif selon l'invention a également été testé avec des produits contenant moins de 0.5% en masse d'asphaltènes :

BP15 : Brut arabe extra light contenant 0.45%masse d'asphaltènes

BP16 : brut Olmelca contenant 0.3%masse d'asphaltènes.

Le tableau 4 rassemble les valeurs S, Sa et So obtenues. Pour chacune des mesures, le coefficient de corrélation $R^2$ de la courbe de précipitation est supérieur à 0,98. Cet exemple démontre que le dispositif selon l'invention permet de déterminer le seuil de floculation de produits noirs mêmes à de très faibles teneurs en asphaltènes.

Tableau 4 Détermination par la méthode de mesure B des valeurs S, Sa et So pour les bruts PB15 et BP16.

| | Méthode de mesure B | | | |
|---|---|---|---|---|
| | S | Sa | So | $R^2$ |
| BP15 | 2.07 | 0.779 | 0.457 | 0,9820 |
| BP16 | 2.33 | 0.802 | 0.46 | 0,9954 |

**Revendications**

1. Dispositif de mesure (1) du seuil de floculation d'un milieu colloïdal par addition de solvant aliphatique, comprenant :

# EP 4 168 781 B1

- au moins une cellule de mesure (10) fonctionnant par transmission optique directe et présentant une chambre de mesure (101) définie par des parois fixes, destinée à recevoir le milieu à l'intérieur de la chambre de mesure, et, associé à chaque cellule de mesure :

    o un émetteur (12) de lumière configuré pour émettre un faisceau lumineux en forme de cône entrant dans la chambre de mesure suivant une direction d'émission,
    o un récepteur (14) de lumière recevant directement le faisceau lumineux sortant de la chambre de mesure,
    ◦ optionnellement un élément optique situé entre l'émetteur et la cellule de mesure,
    o un organe de déplacement motorisé (16) d'un élément choisi parmi l'émetteur, la cellule de mesure et l'élément optique, suivant une direction parallèle à la direction d'émission,

- un système de gestion (20) agencé pour commander l'organe de déplacement motorisé de chaque cellule de mesure pour régler le volume de chaque chambre de mesure traversé par le faisceau lumineux et agencé pour moduler l'intensité lumineuse du faisceau lumineux émis par l'émetteur en faisant varier l'intensité d'un courant continu alimentant l'émetteur de manière à obtenir un signal détectable par le récepteur.

2. Dispositif de mesure (1) selon la revendication 1, **caractérisé en ce que** chaque chambre de mesure (101) présente deux éléments optiques fixes (102, 103) formant des parois opposées, l'émetteur et le détecteur associés étant situés en dehors de la chambre de mesure, optionnellement chaque chambre de mesure (101) est définie par deux éléments optiques, chacun choisi parmi une lame à faces parallèles, une lentille sphérique et une lentille asphérique.

3. Dispositif de mesure (1) selon l'une quelconque des revendications 1 ou 2, **caractérisé en ce que** chaque organe de déplacement motorisé (16) déplace l'émetteur associé, celui-ci étant configuré pour émettre le faisceau lumineux directement sur la chambre de mesure.

4. Dispositif de mesure (1) selon l'une quelconque des revendications 1 à 3, **caractérisé en ce qu'**il comprend au moins un capteur de température (25) et au moins un organe de régulation de température (23, 24) reliés au système de gestion (20) et **en ce que** le système de gestion (20) est agencé pour réguler la température du milieu.

5. Dispositif de mesure (1) selon l'une quelconque des revendications 1 à 4, **caractérisé en ce que** chaque cellule de mesure (10) comprend une entrée (104) et une sortie (105) de fluide et **en ce que** le dispositif de mesure (1) comprend un circuit de fluide (106) associé à chaque chambre de mesure (101) et relié à la sortie (105) de fluide de celle-ci, le circuit de fluide (106) étant équipé d'un organe de mise en circulation du fluide.

6. Dispositif de mesure (1) selon la revendication 5, **caractérisé en ce que** chaque circuit de fluide comprend un ou plusieurs des éléments suivants :

    - au moins un réservoir (110) et au moins une conduite d'injection (109) de liquide reliée à chaque réservoir (110),
    - une enceinte de mélange (113) présentant une entrée et une sortie connectées au circuit de fluide (106),
    - au moins un organe de régulation de température (22, 24).

7. Dispositif de mesure (1) selon la revendication 5 ou 6, **caractérisé en ce que** le circuit de fluide forme une boucle fermée à l'intérieur de laquelle circule le milieu.

8. Dispositif de mesure (1) selon l'une quelconque des revendications 5 à 6, **caractérisé en ce qu'**il comprend des moyens d'injection de liquide en continu, et notamment à débit constant, à l'intérieur du circuit de fluide.

9. Procédé de mesure du seuil de floculation d'un milieu colloïdal par addition de solvant aliphatique mis en œuvre au moyen d'un dispositif de mesure du seuil de floculation selon l'une quelconque des revendications 1 à 8, comprenant les étapes suivantes :

    (i) on introduit le milieu à l'intérieur de la chambre de mesure définie par des parois fixes de la cellule de mesure fonctionnant par transmission optique directe,
    (i1) optionnellement, une étape de dilution dudit milieu avec une quantité prédéterminée de solvant aliphatique préalablement à l'étape (i),
    (ii) on règle automatiquement le volume de la chambre de mesure traversé par le faisceau lumineux, et une intensité lumineuse émise par l'émetteur en faisant varier l'intensité d'un courant continu alimentant l'émetteur, à l'aide du système de gestion de manière à obtenir un signal détectable par le récepteur,

(iii) on détermine le seuil de floculation à l'aide du dispositif de mesure la floculation après addition de la quantité de solvant aliphatique nécessaire à la floculation, optionnellement, le solvant aliphatique est ajouté en continu, et notamment à débit constant, et on procède aux mesures à l'aide du dispositif de mesure alors que le solvant aliphatique est en cours d'addition.

10. Procédé selon la revendication 9 dans lequel l'émetteur émet un faisceau lumineux dans le domaine du NIR et on détermine l'occurrence de la floculation par détermination du pic d'absorption.

11. Procédé selon l'une des revendications 9 à 10, dans lequel on détermine l'occurrence de la floculation à une température prédéterminée réglable.

12. Procédé selon l'une des revendications 9 à 11, dans lequel le milieu comprend des asphaltènes.

13. Procédé selon l'une quelconque des revendications 9 à 12, dans lequel la lumière est constituée de longueurs d'ondes appartenant à un domaine spectral choisi parmi le domaine spectral du proche infra-rouge et le domaine spectral de l'infra-rouge.

14. Procédé de détermination de la stabilité d'un mélange comprenant des asphaltènes par mise en œuvre successive au moins deux fois du procédé selon l'une des revendications 9 à 13 sur un milieu contenant le mélange et une quantité donnée de solvant aromatique, à des taux de dilution différents, optionnellement, le couple solvant aromatique/-solvant aliphatique utilisé est le couple toluène/n- heptane.

## Patentansprüche

1. Messvorrichtung (1) zum Messen der Flockungsschwelle eines kolloidalen Mediums durch Zugabe eines aliphatischen Lösungsmittels, umfassend:

- mindestens eine Messzelle (10), die durch direkte optische Übertragung betrieben wird und eine durch feste Wände definierte Messkammer (101) aufweist, die dazu vorgesehen ist, das Medium im Inneren der Messkammer zu empfangen, und, zugeordnet zu jeder Messzelle:

o einem Lichtsender (12), der konfiguriert ist, um ein in die Messkammer eintretendes Lichtbündel in Form eines Kegels entlang einer Sendungsrichtung auszusenden,
o einem Lichtempfänger (14), der das aus der Messkammer austretende Lichtbündel direkt empfängt,
o optional ein optisches Element, das zwischen dem Sender und der Messzelle angeordnet ist,
o ein motorisiertes Stellglied (16) eines Elements, das aus dem Sender, der Messzelle und dem optischen Element ausgewählt ist, entlang einer Richtung parallel zur Sendungsrichtung,

- ein Verwaltungssystem (20), das dazu ausgelegt ist, das motorisierte Stellglied jeder Messzelle zu steuern, um das Volumen jeder vom Lichtbündel durchquerten Messkammer zu regeln, und das dazu ausgelegt ist, die Lichtstärke des vom Sender ausgesendeten Lichtbündels durch Variieren der Stärke eines den Sender speisenden Gleichstroms zu modulieren, sodass ein vom Empfänger detektierbares Signal erhalten wird.

2. Messvorrichtung (1) nach Anspruch 1, **dadurch gekennzeichnet, dass** jede Messkammer (101) zwei feste optische Elemente (102, 103) aufweist, die gegenüberliegende Wände bilden, wobei der Sender und der Detektor, die zugeordnet sind, außerhalb der Messkammer angeordnet sind, optional jede Messkammer (101) durch zwei optische Elemente definiert ist, die jeweils aus einer Lamelle mit parallelen Flächen, einer sphärischen Linse und einer asphärischen Linse ausgewählt sind.

3. Messvorrichtung (1) nach einem beliebigen der Ansprüche 1 oder 2, **dadurch gekennzeichnet, dass** jedes motorisierte Stellglied (16) den zugeordneten Sender verstellt, wobei dieser konfiguriert ist, um das Lichtbündel direkt zur Messkammer auszusenden.

4. Messvorrichtung (1) nach einem beliebigen der Ansprüche 1 bis 3, **dadurch gekennzeichnet, dass** sie mindestens einen Temperatursensor (25) und mindestens ein mit dem Verwaltungssystem (20) verbundenes Temperaturregelungsorgan (23, 24) umfasst, und dass das Verwaltungssystem (20) zur Regelung der Temperatur des Mediums ausgelegt ist.

**5.** Messvorrichtung (1) nach einem beliebigen der Ansprüche 1 bis 4, **dadurch gekennzeichnet, dass** jede Messzelle (10) einen Eingang (104) und einen Ausgang (105) für Fluid umfasst, und dass die Messvorrichtung (1) einen jeder Messkammer (101) zugeordneten und mit deren Fluidausgang (105) verbundenen Fluidkreislauf (106) umfasst, wobei der Fluidkreislauf (106) mit einem Organ zum Umwälzen des Fluids ausgestattet ist.

**6.** Messvorrichtung (1) nach Anspruch 5, **dadurch gekennzeichnet, dass** jeder Fluidkreislauf eines oder mehrere der folgenden Elemente umfasst:

- mindestens einen Behälter (110) und mindestens eine mit jedem Behälter (110) verbundene Einspritzleitung (109) für Flüssigkeit,
- ein Mischgefäß (113), das einen Eingang und einen Ausgang aufweist, die an den Fluidkreislauf (106) angeschlossen sind,
- mindestens ein Temperaturregelungsorgan (22, 24).

**7.** Messvorrichtung (1) nach Anspruch 5 oder 6, **dadurch gekennzeichnet, dass** der Fluidkreislauf eine geschlossene Schleife bildet, in deren Innerem sich das Medium umwälzt.

**8.** Messvorrichtung (1) nach einem beliebigen der Ansprüche 5 bis 6, **dadurch gekennzeichnet, dass** sie Mittel zur kontinuierlichen Einspritzung von Flüssigkeit, insbesondere mit konstanter Durchflussmenge, in das Innere des Fluidkreislaufs umfasst.

**9.** Verfahren zur Messung der Flockungsschwelle eines kolloidalen Mediums durch Zugabe eines aliphatischen Lösungsmittels, das durch eine Messvorrichtung zum Messen der Flockungsschwelle eines kolloidalen Mediums nach einem beliebigen der Ansprüche 1 bis 8 eingesetzt wird und die folgenden Schritte umfasst:

(i) Einführen des Mediums in das Innere der Messkammer, die durch feste Wände der durch direkte optische Übertragung betriebenen Messzelle definiert ist,
(i1) optional einen Schritt zum Verdünnen des Mediums mit einer vorbestimmten Menge eines aliphatischen Lösungsmittels vor dem Schritt (i),
(ii) automatisches Regeln des Volumens der vom Lichtbündel durchquerten Messkammer und einer vom Sender ausgesendeten Lichtstärke durch Variieren der Stärke eines den Sender speisenden Gleichstroms mit Hilfe des Verwaltungssystems, sodass ein vom Empfänger detektierbares Signal erhalten wird,
(iii) Bestimmen der Flockungsschwelle mit Hilfe der Messvorrichtung nach Zugabe der zur Flockung erforderlichen Menge an aliphatischem Lösungsmittel, optional wird das aliphatische Lösungsmittel kontinuierlich beigemengt, und insbesondere mit konstanter Durchflussmenge, und Durchführen der Messungen mit Hilfe der Messvorrichtung bei laufender Zugabe des aliphatischen Lösungsmittels.

**10.** Verfahren nach Anspruch 9, wobei der Sender ein Lichtbündel im NIR-Bereich aussendet und das Auftreten der Flockung durch Bestimmen des Absorptionspeaks bestimmt wird.

**11.** Verfahren nach einem der Ansprüche 9 bis 10, wobei das Bestimmen des Auftretens der Flockung bei einer vorbestimmten, regelbaren Temperatur erfolgt.

**12.** Verfahren nach einem der Ansprüche 9 bis 11, wobei das Medium Asphaltene umfasst.

**13.** Verfahren nach einem beliebigen der Ansprüche 9 bis 12, wobei das Licht aus Wellenlängen besteht, die zu einem Spektralbereich gehören, der aus dem Spektralbereich des nahen Infrarots und dem Spektralbereich des Infrarots ausgewählt ist.

**14.** Verfahren zur Bestimmung der Stabilität einer Mischung, die Asphaltene umfasst, durch mindestens zweimaligen sukzessiven Einsatz des Verfahrens nach einem der Ansprüche 9 bis 13 an einem die Mischung und eine bestimmte Menge eines aromatischen Lösungsmittels enthaltenden Medium bei unterschiedlichen Verdünnungsgraden, wobei optional das verwendete Paar aromatisches Lösungsmittel/aliphatisches Lösungsmittel das Paar Toluol/n-Heptan ist.

**Claims**

1.  A measurement device (1) for measuring the threshold for flocculation of a colloidal medium by addition of aliphatic solvent, comprising:

    - at least one measurement cell (10) operating by direct optical transmission and having a measurement chamber (101) defined by fixed walls, intended to receive the medium inside the measurement chamber, and, associated with each measurement cell:

        o an emitter (12) of light, configured to emit a cone-shaped light beam entering into the measurement chamber along a direction of emission,
        o a receiver (14) of light directly receiving the light beam coming out from the measurement chamber,
        o optionally, an optical element located between the emitter and the measurement cell,
        o a motor-operated movement member (16) for an element chosen from the emitter, the measurement cell and the optical element, along a direction parallel to the direction of emission,

    - a management system (20) arranged to control the motor-operated movement member of each measurement cell to adjust the volume of each measurement chamber letting through the light beam and arranged to modulate the intensity of the light of the light beam emitted by the emitter by varying the intensity of a direct current which supplies the emitter so as to obtain a signal which can be detected by the receiver.

2.  The measurement device (1) according to claim 1, **characterized in that** each measurement chamber (101) has two fixed optical elements (102, 103) forming opposite walls, the associated emitter and detector being located outside the measurement chamber, optionally each measurement chamber (101) being defined by two optical elements, each chosen from a plane parallel plate, a spherical lens and an aspherical lens.

3.  The measurement device (1) according to any of claims 1 or 2, **characterized in that** each motor-operated movement member (16) moves the associated emitter, the emitter being configured to emit the light beam directly onto the measurement chamber.

4.  The measurement device (1) according to any of claims 1 to 3, **characterized in that** same comprises at least one temperature sensor (25) and at least one temperature regulation member (23, 24) connected to the management system (20) and **in that** the management system (20) is arranged to adjust the temperature of the medium.

5.  The measurement device (1) according to any of claims 1 to 4, **characterized in that** each measurement cell (10) comprises a fluid inlet (104) and a fluid outlet (105) and **in that** the measurement device (1) comprises a fluid circuit (106) associated with each measurement chamber (101) and connected to the fluid outlet (105) of same, the fluid circuit (106) being equipped with a fluid circulating member.

6.  The measurement device (1) according to claim 5, **characterized in that** each fluid circuit comprises one or a plurality of the following elements:

    - at least one tank (110) and at least one liquid injection pipe (109) connected to each tank (110),
    - a mixing chamber (113) having an inlet and an outlet connected to the fluid circuit (106),
    - at least one temperature regulation member (22, 24).

7.  The measurement device (1) according to claim 5 or 6, **characterized in that** the fluid circuit forms a closed loop inside of which the medium circulates.

8.  The measurement device (1) according to any of claims 5 to 6, **characterized in that** said measurement device comprises means of continuous injection of a liquid, in particular at constant flow rate, inside the fluid circuit.

9.  A method for measuring the threshold for flocculation of a colloidal medium by addition of aliphatic solvent used by a measurement device for measuring the threshold for flocculation according to any of claims 1 to 8, comprising the following steps:

    (i) the medium is introduced into the measurement chamber defined by fixed walls of the measurement cell operating by direct optical transmission,

(i1) optionally, a step of dilution of said medium with a predetermined amount of aliphatic solvent prior to step (i),

(ii) automatic adjustment of the volume of the measurement chamber letting through the light beam, and of an intensity of the light emitted by the emitter by varying the intensity of a direct current which supplies the emitter using the management system so as to obtain a signal which can be detected by the receiver,

(iii) the threshold for flocculation is determined using the measurement device for the measurement of the flocculation after the addition of the amount of aliphatic solvent necessary for flocculation, and optionally the aliphatic solvent is continuously added and in particular at constant flow rate, and measurements are made using the measurement device while the aliphatic solvent is being added.

10. The method according to claim 9 in which the emitter emits a light beam in the **NIR** domain and the occurrence of flocculation is determined by the determination of the absorption peak.

11. The method according to one of claims 9 to 10, wherein the occurrence of flocculation is determined at an adjustable predetermined temperature.

12. The method according to one of claims 9 to 11, wherein the medium comprises asphaltenes.

13. The method according to any of claims 9 to 12, wherein the light is composed of wavelengths belonging to a spectral domain chosen from the near infra-red spectral domain and the infra-red spectral domain.

14. A method for determining the stability of a mixture comprising asphaltenes by implementing successively, at least twice, the method according to one of the claims 9 to 13 on a medium containing the mixture and a given amount of aromatic solvent, at different dilution rates and optionally the aromatic solvent/aliphatic solvent pair used is the toluene/n-heptane pair.

**COURBE DE PRECIPITATION D'UN PRODUIT NOIR**

Fig. 1

Figure 2

Figure 3a

Figure 3b

Figure 4

Fig. 5a

Fig. 5b

Fig. 5c

Fig. 6a

Fig. 6b

Fig. 6c

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2596522 A
- US 4628204 A
- DE 3714755 A1
- US 2010053622 A1
- WO 2005003754 A2
- EP 1751518 B1